# EUROPEAN PATENT APPLICATION

(11) **EP 2 727 561 A1**
(43) Date of publication of application: **07.05.2014**
(21) Application number: 13275273.4
(22) Date of filing: 31.10.2013
(51) Int. Cl.: A61F 2/91, A61F 2/915, A61F 2/06, A61F 2/07, A61F 2/82

(54) **Fixation process for nesting stents**

(30) Priority: 31.10.2012 US 201261720863 P; 30.10.2013 US 201314066964
(71) Applicant: Cook Medical Technologies LLC, Bloomington, IN 47404 (US)
(72) Inventor: Hadley, Richard D., Otterbein, IN Indiana 47970 (US); Sisken, Richard B., West Lafayette, IN Indiana 47906 (US)
(74) Representative: Williams Powell

(57) **Abstract**

A stent graft device that requires an anchor setting procedure to prevent migration of the stent, such as an endovascular anchor stent device used for treatment of an abdominal aortic aneurism, can be anchored to a blood vessel by electrosurgically disrupting portions of the blood vessel *in situ* to affix struts of the stent graft device to the vessel wall, replacing barbs which have been used in such devices. The stent graft device can have a wire frame and struts or a cannula stent frame body with struts cut from the cannula. The struts (13, 38) can be provided with openings (47) which are filled with extracellular matrix material, which encourages and speeds ingrowth of the struts (13, 38) in the vessel wall.

## Description

### FIELD OF THE INVENTION

This invention relates generally to devices for use in medical procedures, and more particularly, to nesting stents of devices such as endovascular graft devices implanted in body cavities, *e.g.,* human blood vessels such as the aorta, in order to treat abdominal aortic aneurisms.

### BACKGROUND OF THE INVENTION

The aorta takes blood from the heart initially upwards, then arches backward and flows downward in front of the spinal column until it divides into the right and left iliac arteries. Some arteries branch off directly from the aorta to various parts of the body, such as the renal arteries that feed blood to the kidneys. The two iliac arteries take blood from aorta to the lower portions of the body.

In persons with iliac arteries narrowed by arterial disease (e.g., atherosclerosis), an aortic aneurysm (dilation of the aorta) may occur below the renal arteries, shortly before the division of the aorta into the iliac arteries. If the aneurysm is untreated, the aorta weakens and bulges outward like a balloon. If the aneurysm is severe enough that it ruptures, blood flows freely into the abdomen instead of into the two iliac arteries, and the patient is at high risk of rapid death due to internal bleeding. A ruptured aortic aneurysm is a life-threatening emergency, and surgery must be quickly performed in order to save the patient's life. An unruptured symptomatic aneurysm is not quite as dangerous, but still requires prompt surgery to repair the aneurysm before rupture.

In recent years treatment of aneurysms has been performed prior to aneurysm rupture and has included the use of stent grafts that are implanted within the vascular system with minimally invasive surgical procedures and that include one or more stents affixed to graft material. (As used herein, "stent" refers to a device inserted into a natural passage in the body, such as an artery, to reinforce a weakened portion or to prevent or counteract a flow constriction. "Stent" sometimes refers to the structural portion, or frame body, of the device; "graft", to a biocompatible material that covers the frame; and "stent graft", to the composite structure.)

Stent grafts are secured at a treatment site by endovascular insertion utilizing introducers and catheters, during or after which they are enlarged radially and remain in place by friction and/or attachment to the vessel wall. In particular, stent grafts are known for use in treating thoracic and abdominal aortic aneurysms where the stent graft at one end defines a single lumen for placement within the aorta and the other end is bifurcated to define two lumens, for extending into the branch arteries. An example of such a stent graft is the Zenith AAA (abdominal aortic aneurism) stent graft sold by Cook Medical Incorporated of Bloomington, Indiana, USA.

Cannula laser cut anchor stents, with incorporated barbs cut in the cannula, are used with endovascular graft devices. These barbs are heat-set outward of the radius of the stent so that they will affix when deployed by the physician into the wall of the blood vessel, to prevent stent migration. Wire stent design anchor stents are also provided with attachment barbs. In either case, fixation is key to the deployment of the stent and affixing the stent in its final nesting location.

The use of barbs, however, introduces potential risks, including difficulty of deployment, due to barbs potentially getting caught in the delivery system; barb fatigue; puncture of the vessel wall by the barbs; cutting and even dissection of the vessel due to puncture; limited repositioning of the stent after deployment; and premature anchoring of the stent during deployment, causing nesting to occur in an unwanted location.

### BRIEF SUMMARY OF THE INVENTION

Aspects of the present invention seek to provide an improved apparatus and method for securing an endovascular prosthesis in a body cavity.

According to an aspect of the present invention, there is provided an apparatus as in claim 1.

According to an aspect of the invention, there is provided a process as in claim 12.

According to an aspect of the invention, there is provided an expandable metal frame stent as in claim 16.

According to an aspect of the invention, there is provided a system for securing an endovascular prosthesis in a body cavity, comprising:
a prosthesis introducer;
at least one expandable bio-compatible frame body disposed on the introducer, the frame body being contractible into a first shape with a smaller diameter for introduction to a vascular site and being radially expandable into a second shape having a larger diameter;
the frame body comprising at least partially electrically conductive struts in contact with and for attachment to an inner wall of the body cavity; and
an insulated electrical conductor, electrically contacting at least one strut of the at least partially electrically conductive struts in contact with the inner wall of the body cavity, the electrical conductor being connectable to radio frequency electrical power, for providing electrical energy for a sufficient time at a sufficient power to affix the strut to the inner wall of the body cavity.

According to an aspect of the invention, there is provided a method for placing a prosthesis to repair a defect in a body cavity, comprising the steps of:
providing a catheter with a preloaded prosthesis comprising at least one expandable tubular bio-compatible at least partially electrically conductive frame body,
   the expandable tubular bio-compatible at least partially electrically conductive frame body being contractible into a first shape with a smaller diameter for introduction into a body cavity and being radially expandable into a second shape having a larger diameter,
   the expandable tubular bio-compatible at least partially electrically conductive frame body having at least partially electrically conductive struts for attachment to an inner wall of the body cavity, and having an insulated electrical conductor, electrically contacting at least one of the at least partially electrically conductive struts for attachment to the body cavity, the insulated electrical conductor being connectable to radio frequency electrical power, for providing electrical energy for a sufficient time at a sufficient power to affix the at least one of the at least partially electrically conductive struts to the inner wall of the body cavity;
introducing the catheter and the preloaded prosthesis into the body cavity and advancing to a treatment site;
releasing the prosthesis from the catheter and expanding the prosthesis so that at least one at least partially electrically conductive strut contacting the electrical conductor is in contact with the body cavity; and
connecting the electrical conductor to radio frequency electrical power for a sufficient time at a sufficient power to affix the at least one at least partially electrically conductive strut to the inner wall of the body cavity.

Preferably, the electrical conductor is subsequently removed.

Preferably, the expandable tubular bio-compatible at least partially electrically conductive frame body is a stent, and the at least partially electrically conductive struts have at least one opening therein for the receipt of extracellular matrix material.

Preferably, the at least one opening comprises a bore having a depth extending all the way through the at least partially electrically conductive struts, and the extracellular matrix material extends through the depth.

Preferably, the expandable tubular bio-compatible at least partially electrically conductive frame body and struts are formed from metal wire.

Preferably, the sufficient time and the sufficient power accomplish a preliminary attachment of the strut to the inner wall of the body cavity, the preliminary attachment being of such strength that the preliminary attachment can be broken without harming the wall of the body cavity to which the prosthesis is attached.

Preferably:
the endovascular prosthesis is preliminarily deployed in a preliminary placement;
an initial sufficient time and an initial sufficient power accomplish a preliminary attachment of a strut to the inner wall of the body cavity;
the preliminary placement of endovascular prosthesis is observed by a physician after the preliminary attachment of the bio-compatible material to the inner wall of the body cavity;
the preliminary attachment is broken;
the endovascular prosthesis is deployed in a new placement;
the new placement of endovascular prosthesis is observed by a physician; and
a second sufficient time and a second sufficient power accomplish a secondary attachment of the strut to the inner wall of the body cavity.

Preferably, the body cavity is an aorta, the initial sufficient time is from about 3 to about 4 seconds, the initial sufficient power is from about 70 to about 80 watts, the second sufficient time is from about 3 to about 4 seconds, and the second sufficient power is from about 95 to about 150 watts.

According to an aspect of the invention, there is provided a fixation process for implanting an at least partially electrically conductive endovascular prosthesis within a body cavity, comprising the steps of:
contacting an electrical conductor that is supplied with radio frequency electrical power to at least a portion of the at least partially electrically conductive endovascular prosthesis;
positioning the at least partially electrically conductive endovascular prosthesis within the body cavity in contact with a wall of the body cavity; and
activating the radio frequency electrical power for sufficient time at sufficient power to affix at least a portion of the at least partially electrically conductive endovascular prosthesis to the wall of the body cavity.

Preferably, the sufficient time and sufficient power accomplish a preliminary attachment of the at least partially electrically conductive endovascular prosthesis to the inner wall of the body cavity, the preliminary attachment being of such strength that the preliminary attachment can be broken without harming the wall of the body cavity in need of reinforcement to which the at least partially electrically conductive endovascular prosthesis is attached.

Preferably:
the endovascular prosthesis is preliminarily deployed in a preliminary placement;
an initial sufficient time and an initial sufficient power accomplish a preliminary attachment of the at least partially electrically conductive endovascular prosthesis to the inner wall of the body cavity;
the preliminary placement of at least partially electrically conductive endovascular prosthesis is observed by a physician after the preliminary attachment to the inner wall of the body cavity;
the preliminary attachment is broken;
the endovascular prosthesis is deployed in a new placement;
the new placement of the endovascular prosthesis is observed by a physician; and
a second sufficient time and a second sufficient power accomplish a secondary attachment of the endovascular prosthesis to the inner wall of the body cavity.

Preferably, the endovascular prosthesis comprises a tubular bio-compatible metallic frame body.

Preferably, the body cavity is an aorta, wherein the initial sufficient time is from about 3 to about 4 seconds, the initial sufficient power is from about 70 to about 80 watts, the second sufficient time is from about 3 to about 4 seconds, and the second sufficient power is from about 95 to about 150 watts.

Embodiments of the present invention provide a method of deployment of a stent graft such as an endovascular anchor stent graft for an endovascular prosthesis (*i.e*., prosthetic device), using radio frequency fixation ("RFF") of struts of the stent graft to the inner walls of the aorta.

More particularly, embodiments of the invention provide a system for securing an endovascular prosthesis in a body cavity, comprising a prosthesis introducer, at least one expandable bio-compatible frame body disposed on the introducer, which is contractible into a first shape with a smaller diameter for introduction to a vascular site and expandable into a second shape having a larger diameter. The frame body has at least partially electrically conductive struts in contact with and for attachment to an inner wall of the body cavity. The system further includes an insulated electrical conductor electrically contacting at least one strut of the at least partially electrically conductive struts in contact with the inner wall of the body cavity, the electrical conductor being connectable to radio frequency electrical power, for providing electrical energy for a sufficient time at a sufficient power to affix the strut to the inner wall of the body cavity.

Embodiments of the invention further provide a method for placing a prosthesis to repair a defect in a body cavity, comprising providing a catheter with a preloaded prosthesis comprising at least one expandable tubular bio-compatible, least a partially electrically conductive frame body. The expandable tubular biocompatible, at least partially electrically conductive frame body being contractible into a first shape with a smaller diameter for introduction into a body cavity, being radially expandable into a second shape having a larger diameter, having at least partially electrically conductive struts for attachment to an inner wall of the body cavity, and having an insulated electrical conductor electrically contacting at least one of the at least partially electrically conductive struts for attachment to the body cavity. The insulated electrical conductor being connectable to radio frequency electrical power, for providing electrical energy for a sufficient time at a sufficient power to affix the at least one of the at least partially electrically conductive struts to the inner wall of the body cavity. The method further includes introducing the catheter and the preloaded prosthesis into the body cavity and advancing to a treatment site, releasing the prosthesis from the catheter and expanding the prosthesis so that at least one at least partially electrically conductive strut contacting the electrical conductor is in contact with the body cavity, and connecting the electrical conductor to radio frequency electrical power for a sufficient time at a sufficient power to affix the at least one at least partially electrically conductive strut to the inner wall of the body cavity.

More generally, embodiments of the invention provide a fixation process for implanting an at least partially electrically conductive endovascular prosthesis within a body cavity, comprising the steps of: contacting an electrical conductor that is supplied with radio frequency electrical power to at least a portion of the at least partially electrically conductive endovascular prosthesis; positioning the at least partially electrically conductive endovascular prosthesis within the body cavity in contact with a wall of the body cavity; and activating the radio frequency electrical power for sufficient time at sufficient power to affix at least a portion of the at least partially electrically conductive endovascular prosthesis to the wall of the body cavity.

Embodiments of the invention also provide an expandable metal frame stent for securing an endovascular prosthesis in a body cavity, the metal frame stent being contractible into a first shape with a smaller diameter for introduction to a vascular site and being radially expandable into a second shape having a larger diameter; the frame stent comprising struts for attachment to an inner wall of the body cavity; at least one strut of the struts having at least one opening in the strut; and extracellular matrix material being disposed in the at least one opening.

### BRIEF DESCRIPTION OF THE DRAWINGS

Embodiments of the invention are described below, by way of example only, with reference to the accompanying drawings.
Figure 1 illustrates an abdominal aortic aneurism and a unitary stent graft assembly, deployed within the aneurism.
Figure 2 illustrates an abdominal aortic aneurism and a bifurcated stent graft assembly, deployed within the aneurism.
Figure 3 is an expanded view of a stent graft assembly as shown in Figure 1, but illustrating in addition the barbs of the prior art devices.
Figures 4-6 illustrate an exemplary cannula cut stent graft frame.

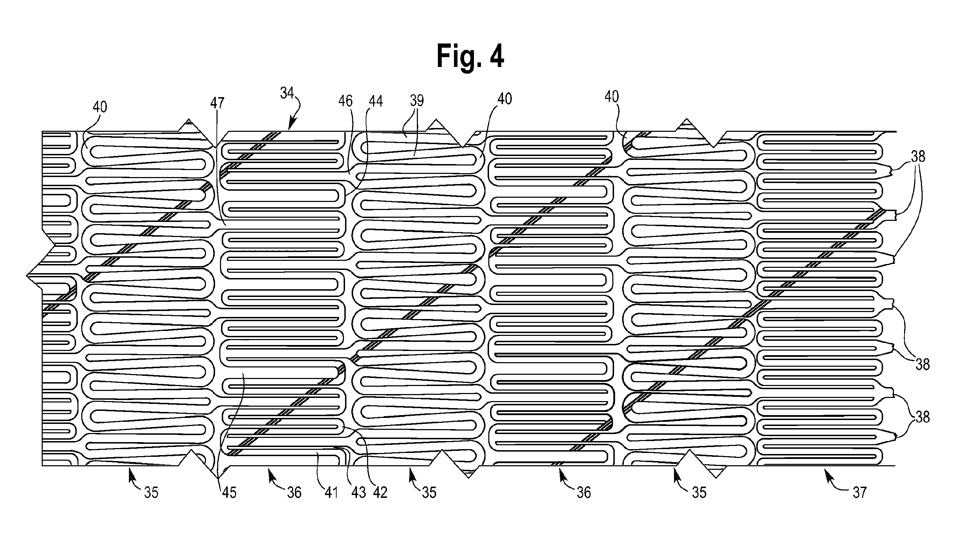

Figure 4 is a flat view of a portion of the exemplary cannula cut stent graft frame, cut from a cylindrical piece of cannula.

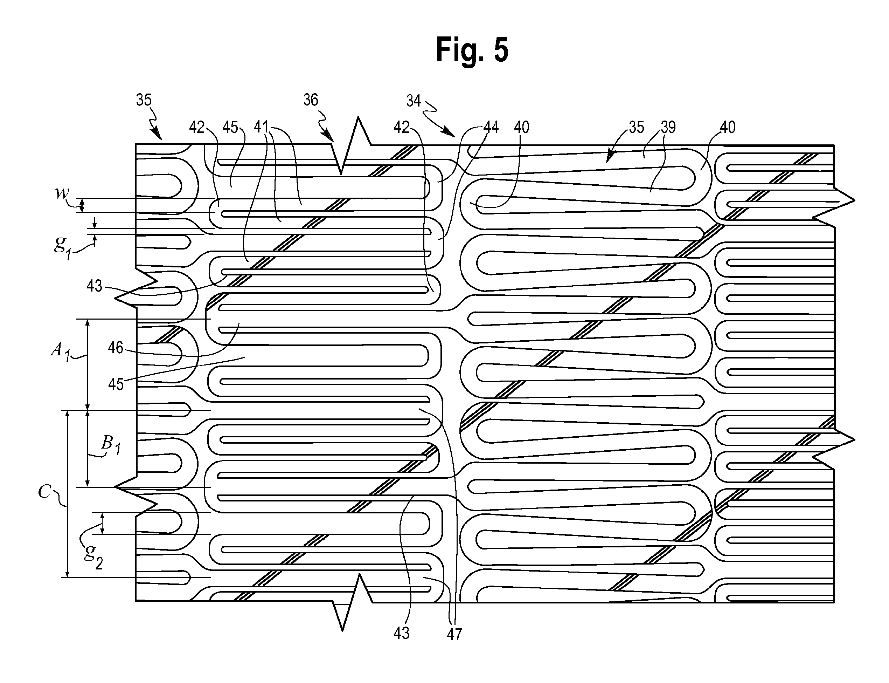

Figure 5 is an enlarged view of the stent of Figure 4.

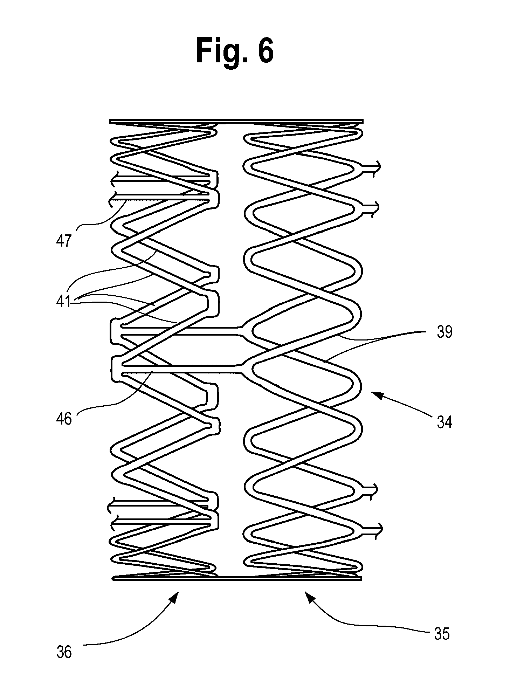

Figure 6 is a side view of the stent of Figure 4, when in an expanded state.

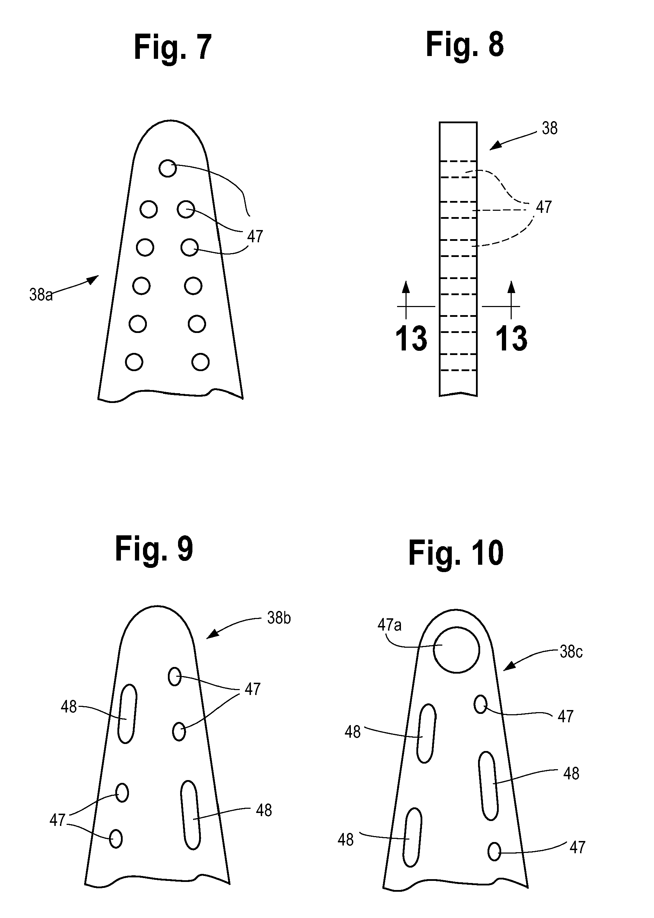

Figures 7 and 9-11 are front views of alternate designs of individual attachment struts.
Figure 8 is a side view of the attachment strut of Figure 7.

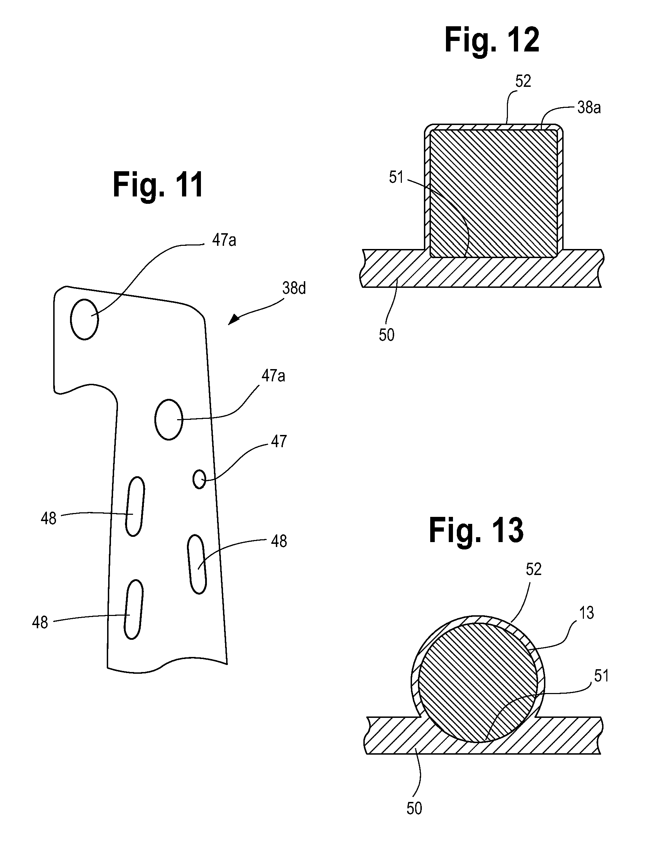

Figure 12 is a view of a cannula of a cannula cut embodiment of the invention, with a parylene coating on the portion of the cannula not intended to contact the vessel wall directly.
Figure 13 is a view of a wire of a wire stent embodiment of the invention, with a parylene coating on the portion of the wire not intended to contact the vessel wall directly.

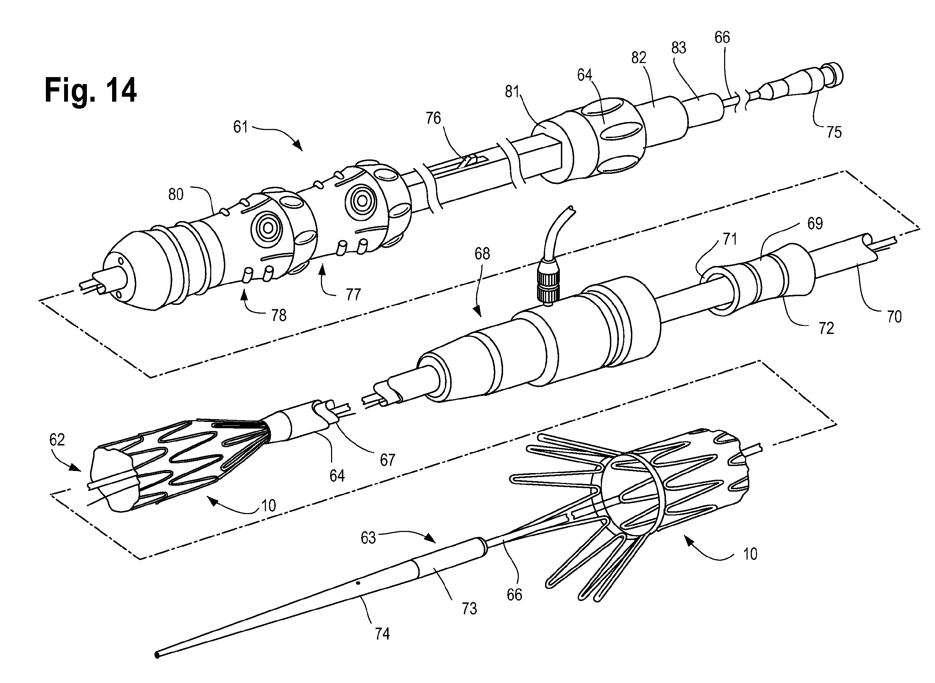

Figure 14 illustrates a delivery system that can be used in or in conjunction with embodiments of the invention.

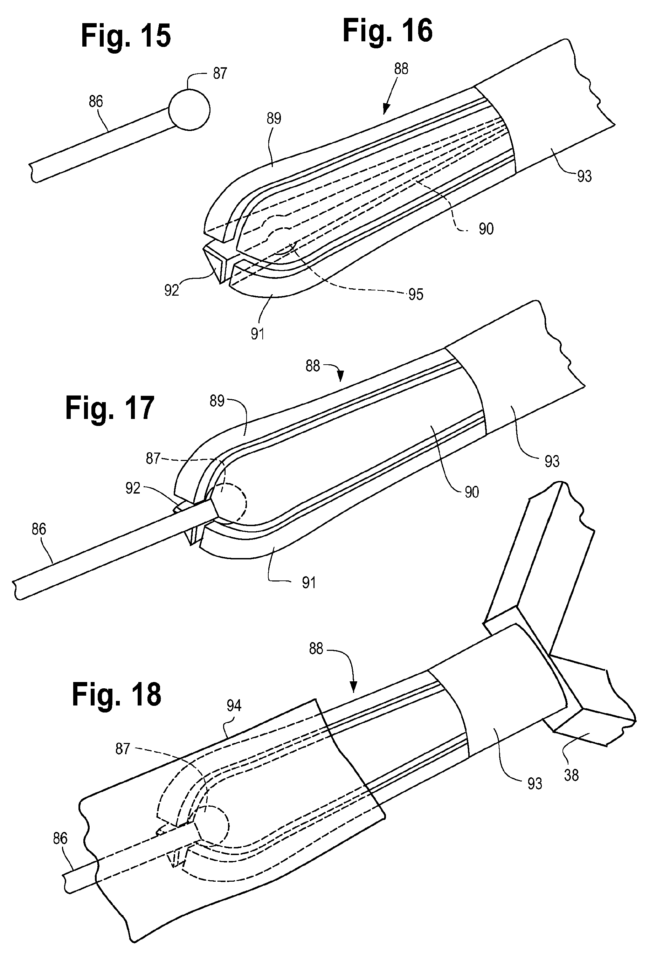

Figures 15-18 illustrate a removable connection to an endovascular device, suitable for use in embodiments of the present invention.

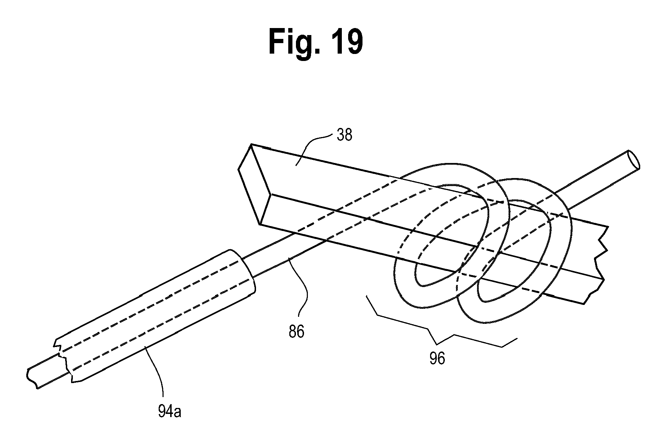

Figure 19 illustrates an alternative attachment mechanism.

### DETAILED DESCRIPTION

The expandable frame body can be a stent. The stent should be at least partially electrically conductive, conveniently by comprising metal. The endovascular prosthesis conveniently can comprise a tubular bio-compatible metallic frame body. Embodiments of the invention can be used with either self-expanding or balloon-expandable stents. Self-expanding stents are more frequently used in the aorta. With either self-expanding or balloon-expandable stents, a balloon can be employed to displace blood when the energy to fixate the stent is applied, to help steer the current from the stent directly into the wall of the aorta.

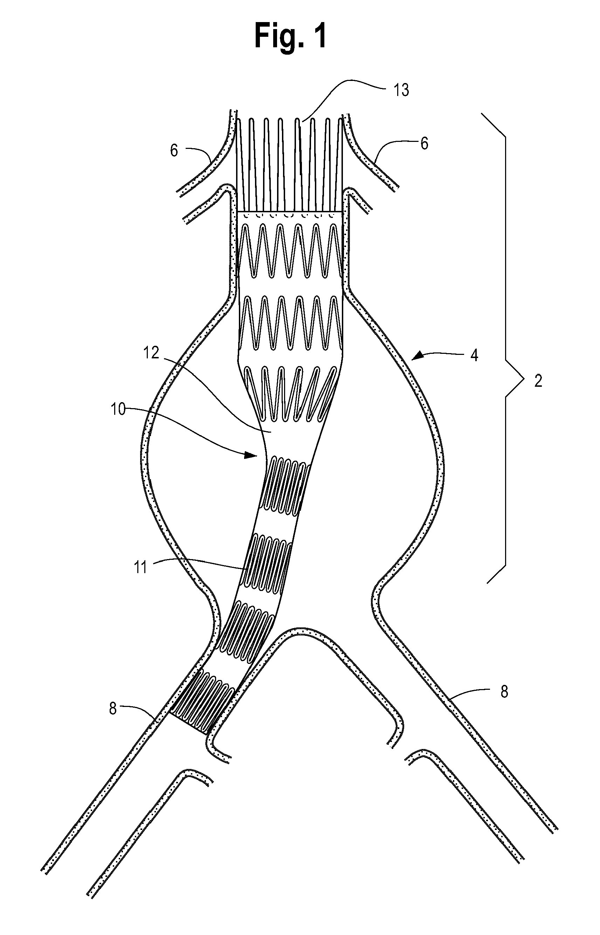

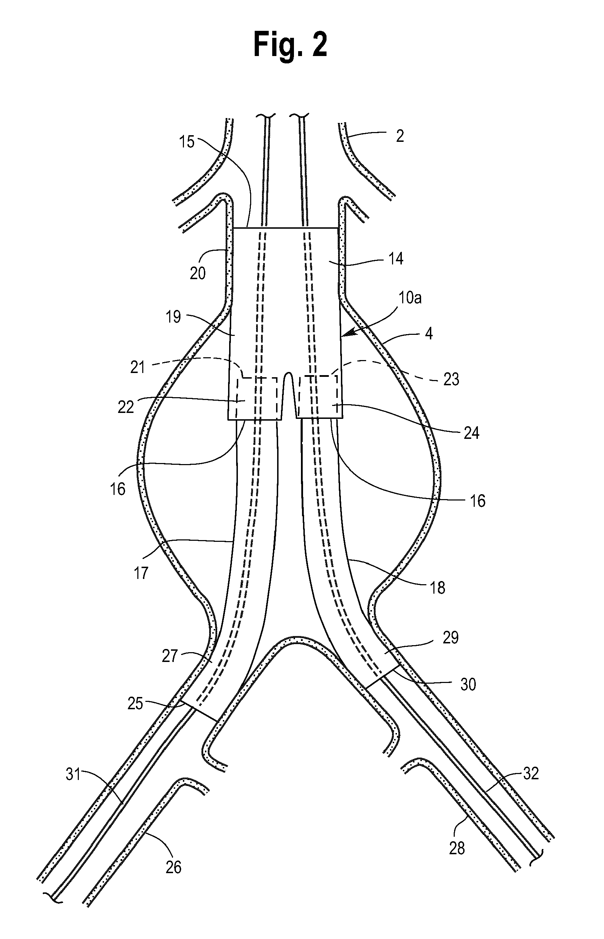

The stents can be either unitary (see Fig. 1) or bifurcated (see Fig. 2), *i.e.,* branched in the lower portion into two sections for extension into the right and left iliac arteries. The stent frame body can be either of a wire-stent design (see Figs. 1 and 13), or of a cannula cut stent design (see Figs. 4-12).

Embodiments of the invention can be used with struts which are either wire extensions from the wire-stent design, or cut extensions of the cannula cut stent design. With a cannula cut stent design, remodelable extracellular matrix ("ECM") material, such as porcine small intestinal submucosa ("SIS"), can be planted in at least one opening in the stent struts to enhance grafting to the vessel wall. The at least one opening preferably extends through the strut, and can comprise a bore having a depth extending all the way through the strut, and wherein the extracellular matrix material extends through the depth. The use of SIS also has the advantage that it can promote tissue growth, and after the initial fixation has taken place, help the aorta wall to grow around the struts of the stent, to better affix the stent in its nesting location.

The stent frame body is constructed with from about 4 to about 14 apices which protrude from each end of the device, depending on the expanded diameter of the device. This is a precise number with a wire-stent design, because they are formed from a single piece of wire. For a cannula-cut stent, the better measure is to count cells around the circumference of a stent. One stent may have open cells at the ends, closed cells between the ends, and different sections of the same stent may have more or fewer cells than another section.

The frame body can, in addition, be provided with at least one covering of bio-compatible material, wherein the covering is adapted to be spread out by the expansion of the tubular frame.

Preferably, the openings for the receipt of extracellular matrix material are approximately circular in shape; and the openings contain extracellular matrix material, preferably porcine small intestinal submucosa. The more openings, the more securely the stent will be affixed to the wall of the aorta; but the number and size of openings are limited by the structure of the stents, which need to be strong enough to maintain their integrity in use. The openings need not be cut all the way through the stents, but it would be advantageous to do so, and for the extracellular matrix material to extend through the depth of the openings. Fibrous tissue can more firmly grasp the stent struts if the tissue goes all the way through the strut.

The disruption to the aorta caused by the passage of electrical current and the ECM works synergistically. That is, the disruption stimulates the ability of the ECM to cause remodeling of the aorta wall. The inside of the aorta wall on older patients is entirely dead tissue, which does not respond to the growth factors present in the ECM.

The ECM is inserted in several ways. The ECM can be vacuum-pressed, after which it is sufficiently rigid that it does not re-hydrate sufficiently quickly to fall out before being implanted. Tiny ribbons of ECM can be sewn into multiple openings. ECM plugs can be glued in place. A slot can be laser cut between two holes that would hold a strip of ECM.

Embodiments of the invention can be adapted to any stent device that requires an anchor setting procedure to prevent migration of the stent, such as an endovascular anchor stent device, of any size or design. Indeed, embodiments of the invention are adaptable to nesting an endovascular prosthesis comprising a tube of bio-compatible material within any body cavity in need of reinforcement.

Energy is provided by a standard electrosurgical unit ("ESU") that can be bipolar (*i.e*., having two electrical probes which can be electrically connected to complete an electrical circuit); or, preferably, monopolar (i.e., having one electrical probe in which electrical energy flows from an exposed active electrode to a target location, which electrical energy dissipates through the patient's body to a passive return current electrode that is externally connected to a suitable location on the patient's body). In the monopolar configuration, the patient's body is part of the return current circuit. The energy provided by the ESU travels through at least one strut to the bodily vessel or other target location, and cauterizes and adheres the tissue coming into contact with the struts, assisted by the ECM, if present.

Typical electrosurgical units have monopolar and bipolar outputs (see R.D. Tucker et al., "The Interaction between electrosurgical generators, endoscopic electrodes, and tissue", Gastrointestinal Endoscopy 38(2): 118-122 (1992)). Electrosurgical units are marketed by Covidien plc, Dublin, Ireland, and Covidien Surgical Solutions Group, Boulder, CO 80301, USA; and are described, for example, in A.G. Harrell et al., "Energy Sources in Laparoscopy", Seminars in Laparoscopic Surgery 11 (3): 201-09 (2004), and in the web site www.valleylab.com. The stent graft assembly is typically deployed within an abdominal aortic aneurism by a seven-step process, as follows:
Step 1. A guidewire is introduced into the groin, and fed through an iliac artery and the aneurism in the aorta, to just below the renal arteries.
Step 2. A delivery tube is advanced in the same path, over the guidewire.
Step 3. The sheath of the delivery tube is pulled back, over the stent graft.
Step 4. The fabric proximal edge is aligned below the renal arteries. Gold (or other radiopaque) markers can be placed on the proximal lip of the fabric to assist in placement.
Step 5. The proximal stent attachment is released.
   a. A top cap is usually provided to contain the top stent and barbs for attachment to the aorta. A trigger wire is usually pulled to release the top cap.
   b. The top cap is advanced to release the stent, which is springloaded and expands to fill the available space when the top cap is advanced.
Step 6. The distal graft attachment is released.
Step 7. The delivery tool is withdrawn.

Embodiments of the invention are concerned with Steps 5 and 6. Instead of having barbs to attach the stent graft to walls of the artery, the graft is attached by disrupting a small portion of the wall of the vessel in which the stent graft is employed, and preferably also by remodelable collagenous materials such as SIS planted in lumens of stent strut holes to enhance grafting to the vessel wall, as described further below.

The stent graft may be delivered into a vessel, duct, or other anatomical site using a suitable deployment system or introducer. An introducer, such as that described in Hartley et al. PCT Publication No. WO 98/53761, the disclosure of which is incorporated by reference, may be used to deploy the stent graft. PCT Publication WO98/53761 describes a deployment system for an endoluminal prosthesis, illustrated in Fig. 14, whereby the prosthesis is radially compressed onto a delivery catheter and is covered by an outer sheath. To deploy the system, the operator slides or retracts the outer sheath over the delivery catheter, thereby exposing the prosthesis. The prosthesis expands outwardly upon removal of the sheath. The operator can directly manipulate the sheath and the delivery catheter, which provides the operator with a relatively high degree of control during the procedure. Further, such delivery devices may be compact and may have a relatively uniform, low-diameter radial profile, allowing for atraumatic access and delivery.

The delivery and deployment device used to deploy the stent graft may optionally include deployment control mechanisms. For example, a proximal control mechanism may releasably retain the proximal end of the stent graft and a distal control mechanism may releasably retain the distal end of the stent graft. The proximal and distal control mechanisms may comprise one or more trigger wires that releasably couple the proximal and distal ends of the stent graft to the delivery catheter. Various prosthesis retention devices, configurations, and methods of use are disclosed in PCT Publication WO 98/53761. While the above-referenced PCT Publication describes one system for delivering and deploying the stent graft, other suitable delivery and deployment systems may be used to deliver a stent or stent-graft manufactured in accordance with the embodiments and techniques described hereinabove.

The electrosurgical unit can have an extended controller and a standard return pad, to accomplish nesting of the stent into the vessel wall electrically during surgery. During the deployment procedure and nesting of the stent graft in the vessel wall, RFF anchors the proximal or distal end of the stent graft in place to prevent unwanted migration of the stent graft. Embodiments of the invention use a delivery system that makes electrical connection from the proximal or distal end of the stent to the ESU, which is operated by a foot or hand control by the physician. At the time of nesting the physician may deploy and reposition the stent if necessary, and anchor the stent to the vessel wall at will and with greater ease and accuracy and with less risk of complications than a barbed stent can pose.

The fixation process allows the physician to affix the proximal or distal end of the stent to the vessel inner wall at will and when the stent is fully deployed, while eliminating the use of conventional barbs for anchoring. Embodiments of the invention allow the physician to reposition the stent at the time of nesting, with the capability of viewing the deployed position before committing to it; allows ease of deployment with no chance of barbs contacting and engaging the vessel wall prematurely; and gives the physician the choice of using disruption anchoring at the time of deployment.

Preferably, the sufficient time and sufficient power accomplish a preliminary attachment of the bio-compatible material to the inner wall of the aorta, the preliminary attachment being of such strength that the preliminary attachment can be broken by relocating the endovascular prosthesis without harming the wall of the aorta to which the endovascular prosthesis is attached. Preferably, the endovascular prosthesis is preliminarily deployed in a preliminary placement; an initial sufficient time and an initial sufficient power accomplish a preliminary attachment of the bio-compatible material to the inner wall of the aorta; the preliminary placement of endovascular prosthesis is observed by a physician after the preliminary attachment of the bio-compatible material to the inner wall of the aorta; the preliminary attachment is broken (if necessary) by relocating the endovascular prosthesis; a new placement of endovascular prosthesis is observed by a physician; and a second sufficient time and a second sufficient power accomplish a secondary attachment of the bio-compatible material to the inner wall of the aorta. For example, the initial sufficient time can be from about 3 to about 4 seconds, the initial sufficient power from about 70 to about 80 watts, the second sufficient time from about 3 to about 4 seconds, and the second sufficient power from about 95 to about 150 watts. If the covering of bio-compatible material is meltable, such as polyester, and no extracellular matrix material is used, the disruption of the contacted portion of the covering of bio-compatible material gently melts the meltable covering of bio-compatible material. Preferably, however, the covering has at least one opening therein for the receipt of extracellular matrix material; at least one opening therein for the receipt of extracellular matrix material contains extracellular matrix material; and the disruption of the contacted portion of the covering of bio-compatible material affixes the bio-compatible material to the inner wall of the aorta.

There are many well-known ways of controlling the stent graft system during deployment, which can be used to reposition the device for final placement. Preferably, the sufficient time and sufficient power accomplish a preliminary attachment of the stent graft to the inner wall of the body cavity in need of reinforcement, the preliminary attachment being of such strength that the preliminary attachment can be broken by relocating the stent-graft construct without harming the wall of the body cavity in need of reinforcement to which the bio-compatible material is attached. Preferably, the tube of bio-compatible material is preliminarily deployed in a preliminary placement; an initial sufficient time and an initial sufficient power accomplish a preliminary attachment of the bio-compatible material to the inner wall of the body cavity in need of reinforcement; the preliminary placement of tube of bio-compatible material is observed by a physician after the preliminary attachment of the bio-compatible material to the inner wall of the body cavity in need of reinforcement; the preliminary attachment may be broken; a new placement of the tube of bio-compatible material may be observed by a physician; and a second sufficient time and a second sufficient power may accomplish a secondary attachment of the bio-compatible material to the inner wall of the body cavity in need of reinforcement.

Preferably, the covering has at least one opening therein for the receipt of extracellular matrix material; at least one opening therein for the receipt of extracellular matrix material contains extracellular matrix material; and the disruption of the contacted portion of the covering of bio-compatible material affixes the bio-compatible material to the inner wall of the body cavity in need of reinforcement.

Turning now to the drawings, Fig. 1 illustrates an aorta 2 afflicted with an abdominal aortic aneurism 4; a unitary stent graft assembly 10, deployed within aneurism 4; and a first embodiment of the invention. Branching off from the aorta 2 are right and left renal arteries 6 and right and left iliac arteries 8. The stent graft assembly 10 has been delivered through the groin (not shown) and an iliac artery 8, and comprises an expandable wire frame body 11; a biocompatible covering 12 over the expandable wire frame body; and top stent 13.

Fig. 2 illustrates a modular bifurcated stent graft 10a, implanted to repair an abdominal aorta aneurysm 4. The modular stent graft 10a comprises a first stent graft component 14 having a proximal end 15 and a distal end 16; a second stent graft component 17, and a third stent graft component 18. The proximal end 15 of the trunk 19 of the first stent graft component 14 is implanted in the proximal implantation site 20 in a non-aneurysmal portion of the abdominal aorta 2. The proximal end 21 of the second stent graft component 17 is connected to the first stent graft component 14 at the ipsilateral docking site 22. The proximal end 23 of the third stent graft component 18 is connected to the first stent graft component 14 at the contralateral docking site 24. The distal end 25 of the second stent graft component 17 is implanted in the undilated portion of the ipsilateral iliac artery 26 at the ipsilateral distal implantation site 27. The distal end 30 of the third stent graft component 18 is implanted in a non-dilated portion of the contralateral iliac artery 28 at contralateral distal implantation site 29.

Continuing with Fig. 2, the ipsilateral catheter guide wire 31 is shown coming up from the ipsilateral femoral artery (not shown), through the ipsilateral iliac artery 26, into the second stent graft component 17, through the ipsilateral docking site 22, and out through the proximal end 15 of the trunk 19. The contralateral catheter guide wire 32 is shown coming up from the contralateral femoral artery (not shown), through the contralateral iliac artery 28, into the third stent graft component 18, through the contralateral docking site 24, and out through the proximal end 15 of the trunk 19.

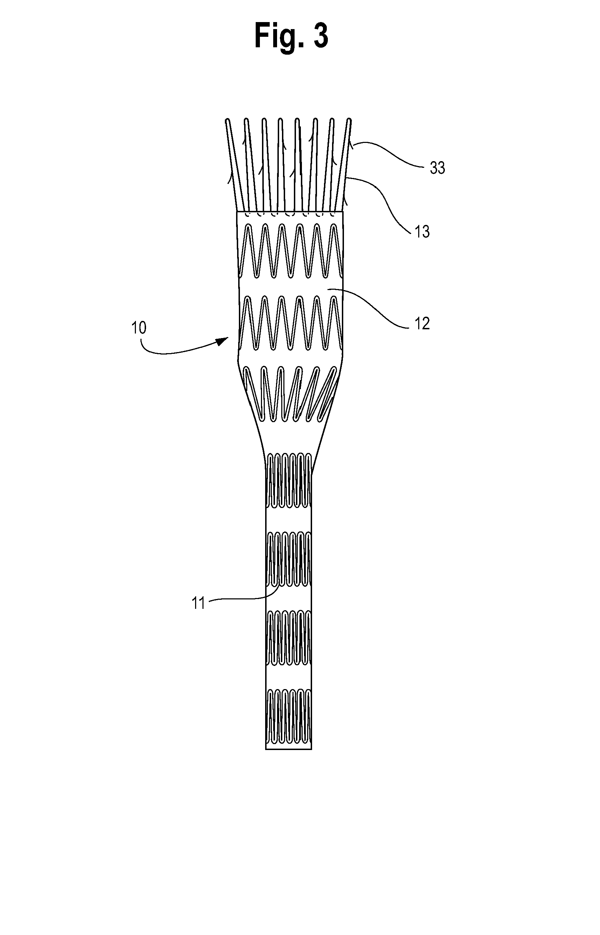

Fig. 3 is an expanded view of a stent graft assembly as shown in Figure 1, but illustrating in addition the barbs 33 of the prior art devices. Fig. 4 is a flat view of a portion of an exemplary cannula cut stent graft frame, cut from a cylindrical piece of cannula. The cannula cut stent graft frame 34 includes a plurality of flexible interconnection segments 35 and higher radial force hoop segments 36, with end cell segment 37 preferably having high hoop strength. End cell segment 37 terminates in top stents 38, shown broken off in Fig. 4, but illustrated more fully in Figs. 7-11 as alternate top stent designs 38a, 38b, 38c and 38d.

The cannula from which the stent graft frame is cut can be Series 304 or similar stainless steel that has application for balloon expandable stents. Alternatively, the cannula can be formed of a nickel-titanium alloy such as nitinol which can be employed for self-expanding stents. These nickel-titanium self-expanding stents normally employ the superelastic properties of nitinol. By way of example, the stent is cut from a piece of cannula when in its compressed condition and then is expanded to its larger diameter expanded state. In the larger diameter expanded state, the nitinol material is heat set so that the stent retains its expanded configuration. The stent is then collapsed and introduced into a guiding catheter for deployment at the placement site.

As depicted in Fig. 4, the flexible interconnection segments 35 have a serpentine configuration that loops back and forth upon itself with spacing between interconnection struts 39, and that varies from one longitudinal end of the segment to the other. Interconnection struts 39 project in spaced apart pairs from respective bights 40 and then, in the unexpanded stent condition, converge at distal ends that each join to other bights 40 to connect with adjacent interconnection strut pairs, thus eventually forming a circumferential band.

The hoop segments 36 also have a serpentine configuration and comprise a series of longitudinal struts 41 that are radially positioned with spacing therebetween that can vary circumferentially. Each pair of adjacent longitudinal struts 41 extends in parallel from a respective bight 42 and struts 41 are closely spaced to define narrow gaps 43, or in parallel from a respective bight 44, more generously spaced apart to define large gaps 45. Distal ends of the longitudinal struts 41 of each pair join to other bights 44 of adjacent strut pairs. Axial tie bars 46 extend from certain bights 44 within large gaps 45 to the right to connect with bights 44 of the adjacent interconnection segment 35 to the right, leaving narrow gaps 43 between the axial tie bar 46 and the adjacent longitudinal struts 41 that may be equal in width to narrow gaps 43; similarly, axial tie bars 47 extend from certain bights 44 within large gaps 45 to the left to connect with bights 40 of another adjacent interconnection segment 35 disposed on the left of hoop segment 12.

Fig. 5 depicts an enlarged view of interconnection segments 35 and hoop segments 36 of cannula cut stent 34 of Fig. 4. In particular, and by way of example, longitudinal struts 41 are approximately 0.7 mm in width (distance "w"), and narrow gaps 43 therebetween are approximately 0.13 mm wide (distance "g₁"). Large gap 45 between selected longitudinal struts 41 is approximately 0.97 mm wide (distance "g₂"). The length and width of the various struts 38, 39, 41 can be varied depending on the diameter of the overall stent. By way of further example, the starting cannula diameter of a stent is approximately 10 mm and may have a metal wall thickness of from about 0.5 to about 1 mm. In this configuration, the hoop segments 36 are connected to the interconnection segments 35 by axial tie bars 46, 47.

In Fig. 5, axial tie bars 46 are spaced circumferentially from each other approximately 7.5 mm (distance "C"). The axial tie bars 46 interconnecting hoop segment 36 with the adjacent interconnection segment 35 extending to the right, are alternated circumferentially with respect to the axial tie bars 46 interconnecting hoop segment 36 with the adjacent interconnection segment 35 to the left. However, as shown, the distance "A₁" between the midlines of axial tie bars 46, 47 connecting right adjacent interconnection segment 35 with left adjacent flexible interconnection segment 35 is 4 mm. This circumferential distance "A₁" includes a large gap 45. Midline distance "B₁" interconnecting adjacent interconnection segments 35, including substantially only narrow gaps 43 of minimal width, is 3 mm. As a result, distance "A₁" is greater than distance "B₁" with non-uniform spacing between circumferential segments. The total of distances "A₁" and "B₁" is approximately 7 mm.

Fig. 6 shows the expanded state of the stent with a configuration as described above and shown in Figs. 4 and 5, with non-uniform spacing between the struts of the hoop segment 36.

Fig. 7 is a front view of one design of an individual top stent 38a, having openings 47 for the receipt of extracellular matrix material, and extracellular matrix material in the openings. Fig. 8 is a side view of the top stent of Figure 7.

Figs. 9, 10 and 11 are front views of alternate designs of individual top stents 38b, 38c and 38d, respectively. Some of the openings 47a (see Figs. 10 and 11) for the receipt of extracellular matrix material can be larger, so as to accommodate a greater amount of extracellular matrix material, and thus allow for temporary attachment with the smaller openings 47, and permanent attachment with the larger openings 47a. Oblong openings 48 can be provided to facilitate movement of the stent graft assembly with a delivery system (see Fig. 14).

Fig. 12 is a cross-sectional view, taken along line 13-13 of Fig. 8, of top stent 38a of a cannula cut embodiment of the invention. Fig. 13 is a cross-sectional view of a top strut 13 of a wire stent embodiment of the invention. In each of Figs. 12 and 13, the top stent 38a or 13 is intended to be applied to a portion of the vessel wall 50, making contact at vessel wall contact area 51. A parylene coating 52 is provided on the portion of the wire top stent 13 or the cannula cut top stent 38a not intended to contact the vessel wall 50 directly, so as to help steer electrical current into tissue rather than blood.

Fig. 14 shows an exemplary delivery system, or introducer, that can be used to deploy the aortic stent graft described above, in perspective view, with the stent graft partially deployed. The introducer is used for deploying an aortic stent graft 10 in an arterial lumen of a patient during a medical procedure. The introducer includes an external manipulation section 61, and a proximal positioning mechanism or attachment region 62. The introducer can also have a distal positioning mechanism or attachment region 63. During a medical procedure to deploy the aortic stent graft 10, the proximal and distal attachment regions 62 and 63 will travel through the arterial lumen to a desired deployment site. The external manipulation section 61, upon which a user acts to manipulate the introducer, remains outside of the patient throughout the procedure.

As illustrated in Fig. 14, the aortic stent graft 10 is retained in a compressed condition by a sheath 64. The sheath 64 can radially compress the aortic stent graft 10 over a distal portion of a thin-walled tube 66. The thin-walled tube 66 is generally flexible and may include metal. A thick-walled tube 67, which can be made of plastic, is coaxial with and radially outside the thin-walled tube 66. The distal end of the thick-walled tube 67 is adjacent to the proximal end of the aortic stent graft 10. The thick-walled tube 67 acts as a pusher to release the stent graft 10 from the introducer during delivery.

The thickness of the wall of thick-walled tube 67 is several times that of the thin-walled tube 66. Preferably, the thick-walled tube 67 is five or more times thicker than the thin-walled tube 66. The sheath 64 is coaxial with, and is positioned radially outside of, the thick-walled tube 67. The thick-walled tube 67 and the sheath 64 extend proximally to the external manipulation region 61, as shown in Fig. 14. The thin-walled tube 66 extends to the proximal end of the introducer. The introducer further includes haemostatic sealing means 68 radially disposed about the sheath 64 and the thick-walled tube 67. The haemostatic sealing means 68 control the loss of blood through the introducer during a procedure.

The introducer may include an aortic stent graft control member 69 as illustrated in Fig. 14. The stent graft control member 69 is disposed on the dilator portion 70 of the external manipulation section 61. During deployment of the aortic stent graft 10, the sheath 64 is withdrawn proximally over the thick-walled tube 67. The haemostatic sealing means 68 generally fits tightly about the sheath 64, resulting in a great amount of friction between the sheath 64 and the thick-walled tube 67. As a result, withdrawal of the sheath 64 over the thick-walled tube 67 can be difficult. In order to overcome the friction, the operator must have a very tight grip on the thick-walled tube 67. Axial positioning of the aortic stent graft 10 may be compromised by the difficulty in gripping the thick-walled tube 67. The control member 69 solves this problem by providing the operator with a better grip on the dilator and by decreasing the force that the operator must exert to control and stabilize the thick-walled tube 67 during withdrawal of sheath 64. The control member 69 is generally tubular and includes an inner dilator facing surface 71 and an outer grip surface 72. The control member 69 is slidably disposed on the thick-walled tube 67 between the haemostatic sealing means 68 and the release wire actuation section to allow the operator to use the control member 69 at any position along the dilator.

The outer grip surface 72 is adapted so that the control member 69 fits the operator's hand comfortably and securely. As such, the outer grip surface 72 may have a diameter that greatly exceeds the diameter of the thick-walled tube 67. The outer grip surface 72 may be generally axially uniform. Alternately, the outer grip surface 72 may be generally axially non-uniform, resulting in a contoured gripping surface. Fig. 14 illustrates a control member 69 having a generally non-uniform outer grip surface 72, wherein the control member 69 is generally shaped like an hour glass. The outer grip surface 72 may include a smooth surface finish, or alternately, the outer grip surface may include a rough or textured surface finish. Rough or textured surface finishes are beneficial because they provide increased surface area contact between the operator and the control member 69, thereby increasing the operator's leverage. Multiple surface finishes may be selected to provide various utilitarian and tactile benefits.

The control member 69 is generally deformable so that when the operator grips the control member 69, the control member 69 compresses against the thick-walled tube 67. The control member 69 transfers the force exerted by the operator to the thick-walled tube 67. The dilator facing surface 71 may include a generally smooth surface. Alternately, the dilator facing surface 71 may have a rough or textured surface. A rough or textured surface may create a more "sticky" or "tacky" contact between the control member 69 and the thick-walled tube 67, thereby increasing the force that is transferred by the operator to the dilator.

The dilator facing surface 71 may include a generally uniform surface. Alternately, the dilator facing surface 71 may include a generally non-uniform surface. For example, the dilator gripping surface 71 may include a plurality of engageable projections that extend radially inward towards the thick-walled tube 67. When the operator grips the control member 69 against the thick-walled tube 67, engageable projections engage the surface of the thick-walled tube 67. Engageable projections increase the surface contact area between the control member 69 and the thick-walled tube 67, thereby increasing the force that the control member 69 transfers from the operator to the thick-walled tube 67. Engageable projections may include any geometric or non-geometric shape. For example, engageable projections may include "O" shapes, lines, dashes, "V" shapes, or the like.

The distal attachment region 63 includes a retention device 73. The retention device 73 holds the distal end of the aortic stent graft in a compressed state. The retention device 73 has at its distal end a long tapered flexible extension 74. The flexible extension 74 includes an internal longitudinal aperture which facilitates advancement of the tapered flexible extension 74 along a previously inserted guidewire. The longitudinal aperture also provides a channel for the introduction of medical reagents. For example, it may be desirable to supply a contrast agent to allow angiography to be performed during placement and deployment phases of the medical procedure.

The distal end of the thin-walled tube 66 is coupled to the flexible extension 74. The thin-walled tube 66 is flexible so that the introducer can be easily advanced. The thin-walled tube 66 extends proximally through the introducer to the manipulation section 61, terminating at a connection means 75. The thin-walled tube 66 is in mechanical communication with the flexible extension, allowing the operator to axially and rotationally manipulate the distal attachment region 63 with respect to the aortic stent graft 10. The connection means 75 is adapted to accept a syringe to facilitate the introduction of reagents into the thin-walled tube 66. The thin-walled tube 66 is in fluid communication with the flexible extension 74, which provides for introduction of reagents through the aperture into the arterial lumen.

The trigger wire release actuation section of the external manipulation section 61 includes an elongate body 76. Distal and proximal trigger wire release mechanisms 77, 78 are disposed on the elongate body 76. End caps are disposed on proximal and distal ends of the elongate body 76. End caps include longitudinally-facing, laterally opposed surfaces defining distal and proximal stops 80, 81. Distal and proximal trigger wire release mechanisms 77, 78 are slidably disposed on the elongate body 76 between distal and proximal stops 80, 81. Distal and proximal stops 80, 81 retain the distal and proximal trigger wire release mechanisms 77, 78 on the elongate body 76. The actuation section includes a locking mechanism for limiting the axial displacement of trigger wire release mechanisms 77, 78 on the elongate body 76.

Referring to the external manipulation section 61, a pin vise 82 is mounted onto the proximal end of the elongate body 76. The pin vise 82 has a screw cap 83. When screwed in, the vise jaws clamp against (engage) the thin-walled metal tube 66. When the vise jaws are engaged, the thin-walled tube 66 can only move with the body 76, and hence the thin-walled tube 66 can only move with the thick-walled tube 67. With the screw cap 83 tightened, the entire assembly can be moved as one with respect to the sheath 64. The self-expanding stent 10 then expands upon its release from the introducer, as shown in Fig. 14.

Figures 15-18 illustrate a removable connection to an endovascular device suitable for use in embodiments of the present invention. The wire itself is shown in Figure 15. Wire 86, which may be stainless steel, nitinol, MP35-N (a nickel-cobalt base alloy having high strength, toughness, ductility and corrosion resistance), tungsten, or similar material, serves to transmit electrical power to the stent 38 (not shown in Figure 15, see Figure 18) which is to be electrosurgically attached to the vessel. Wire 86 is preferably small in diameter, perhaps 0.075 mm, and terminates at ball 87, to which wire 86 is welded or brazed. Ball 87 is preferably as small as is practical, perhaps 0.15 mm in diameter.

Figure 16 illustrates cage 88, which encloses ball 87 (not shown in Figure 16, see Figure 17). Cage 88 can be constructed through laser machining or wire electrical discharge machining ("EDM"), and includes expanding leaves 89-92 (with concavity 95 to match ball 87) which are attached to base 93, which in turn is attached to stent 38 (not shown in Figure 16, see Figure 18). Alternatively, the leaves 89-92, base 93 and stent 38 may all be machined from one piece. In this case, the base 93 may not be necessary.

Figures 17 and 18 illustrate how cage 88 encloses ball 87, and how wire 86, ball 87 and cage 88 are covered by insulating sheath 94, respectively. Insulating sheath 94 may be polyethylene, polytetrafluoroethylene (PTFE), perfluoroalkoxy (PFA, a polymer having the formula - (CF₂ - CF₂)ₙ - (CF₂ - CFO - CF₃)ₘ -), or a similar material. Wire 86, covered by sheath 94, runs back through the introducer system and through the introducer handle, where wire 87 connects to the current supply wire of a standard electrosurgical unit ("ESU"). A handle is connected to insulating sheath 94 so that insulating sheath 94 can be longitudinally withdrawn relative to wire 86. Prior to withdrawing insulating sheath 94, ball 87 is securely held in cage 88 by insulating sheath 94. With insulating sheath 94 in place, and with cage 88 attached to stent 38, electrical current is provided to attach stent 38 to the vessel wall. After stent 38 is attached to the vessel, wire 86 may be used to pull directly on the stent 38 to gauge the strength of attachment of the stent to the vessel wall. When disconnection of wire 86 is desired, insulating sheath 94 is retracted with respect to ball 87, allowing the leaves 89-92 of cage 88 to open and allowing wire 86 to be withdrawn. Cage 88, with leaves 89-92 open, can then be allowed to remain in the patient, or cage 88 can biodegrade if it were machined from a magnesium alloy.

Figure 19 provides an alternative attachment mechanism wherein wire 86 wraps around stent 38 instead of mating with a ball and cage. Several turns of wire 86 (spiral 96) provide a secure joint. In this alternative version, wire 86 should be larger (perhaps 0.20 mm in diameter, depending on the alloy selected), so that when removal of wire 86 is desired, wire 86 can be untwisted from the stent. Insulating sheath 94a is similar to insulating sheath 94 in the embodiment illustrated in Figures 15-18, but clearance between wire 86 and insulating sheath 94a is not required as wire 86 and insulating sheath 94a do not need to move with respect to each other. This allows insulating sheath 94a to be heat-shrunk tubing. Insulating sheath 94a is still needed to perform the insulating function, however, preventing current from straying into places in the body where it is not desired, as is the case with insulating sheath 94 in the embodiment illustrated in Figures 15-18. The operator handle at the delivery system end of wire 86 is somewhat simpler, needing only to be easily rotatable by hand. It is also possible to simply weld wire 86 to stent 38, nicking or crimping the wire to provide a location whereby rotation of wire 86 and sheath 94a will cause metal fatigue and disconnection at the nick or crimped location.

Embodiments of the invention use a standard ESU with a bipolar or preferably a monopolar electrical circuit. Such devices are known in the art; see for example Van Wyk et al. U.S. Patent 7,566,333, the disclosure of which is incorporated by reference in its entirety. An ESU with a monopolar patient connection has an active electrode that supplies radio frequency electrical power. The frame body of the endovascular prosthesis has at least partially electrically conductive struts for attachment to an inner wall of the body cavity; and an insulated electrical conductor, which is the current supply wire, electrically connected to at least one strut of the endovascular prosthesis. The insulated electrical conductor can be, for example, a nitinol pull-wire. The pull-wire can be similar to pull-wires known in the art, that release individual portions of stents, but it is electrically insulated over most of its length, only the ends of the wire being uninsulated. Suitable insulating materials include Parylene HT® and polytetraflouroethylene shrink tube. Because the current that will flow through the pull-wire is low, a wire 0.25 mm in diameter is sufficient. One end is detachably electrically connected to at least one strut of the endovascular prosthesis, and maintains electrical contact with the stent during delivery and positioning of the stent graft; the other end is, after positioning of the stent, connected to the ESU active electrode when the stent is positioned to be affixed to the inner wall of the body cavity. The wire can be looped through two openings in the stent and be removed by being pulled straight out. Or, the wire can be wrapped around the stent in a manner that allows removal by unscrewing the wrapped wire prior to pulling the wire out. Or, the wire could be wrapped around a connection termination point that is similar to a barb, but stays in the cylinder of the stent struts.

A.G. Harrell et al., in "Energy Sources in Laparoscopy," Seminars in Laparoscopic Surgery 11 (3): 201-09 (2004), further describe bipolar and monopolar electrosurgical units. Monopolar electrosurgical units produce current on the order of 300 to 500 kHz, comparable to the 550 kHz to 1600 kHz frequencies used for radio transmission. At such frequencies, the current will not cause fibrillation, and the energy is dissipated as heat in the tissue near to the electrode, which can be used for cutting or ablating tissue. Monopolar electrosurgical units can also be used to generate heat for other purposes.

Monopolar electrosurgical units can be operated in a cutting mode in which the current produced is represented by a continuous sine wave, unmodulated and undamped. The current is of high frequency and high voltage, which results in a rapid temperature rise with explosive vaporization. The lateral thermal spread and depth of necrosis are minimal, but there is little coagulation for the purpose of hemostasis. Monopolar electrosurgical units can also be operated in a coagulation mode, in which the unit produces short bursts of radiofrequency sine waves with pauses between the short bursts. The percentage of time that the radiofrequency is on is described as the duty cycle. In coagulation mode, the current is typically on 6% of the time and off 94% of the time. This waveform provides good hemostasis, but does not cut well.

Most monopolar electrosurgical units provide a blend setting, which provides both cutting and coagulating effects at the same time. On some units, blend 1 is a 50% duty cycle, and blend 2 is a 40% duty cycle. A blend allows for both cutting and hemostasis, the amount of the latter being determined by the specific blend mode.

In certain embodiments, one or more graft elements will comprise a remodelable material. Particular advantage can be provided by devices that incorporate a remodelable collagenous material. Such remodelable collagenous materials, whether reconstituted or naturally-derived, can be provided, for example, by collagenous materials isolated from a warm-blooded vertebrate, especially a mammal. Such isolated collagenous material can be processed so as to have remodelable, angiogenic properties and promote cellular invasion and ingrowth. Remodelable materials may be used in this context to stimulate ingrowth of adjacent tissues into an implanted construct such that the remodelable material gradually breaks down and becomes replaced by new patient tissue so as to generate a new, remodeled tissue structure.

Suitable remodelable materials can be provided by extracellular matrix (ECM) materials possessing biotropic properties. For example, suitable collagenous materials include ECM materials such as those comprising submucosa, renal capsule membrane, dermal collagen, dura mater, pericardium, fascia lata, serosa and peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. Collagenous matrices comprising submucosa (potentially along with other associated tissues) useful in embodiments of the present invention can be obtained by harvesting such tissue sources and delaminating the submucosa-containing matrix from smooth muscle layers, mucosal layers, and/or other layers occurring in the tissue source. For additional information as to some of the materials useful in embodiments of the present invention, and their isolation and treatment, reference can be made, for example, to U.S. Patent Nos. 4,902,508, 5,554,389, 5,993,844, 6,099,567 and 6,206,931, the disclosures of which are incorporated by reference.

Remodelable ECM tissue materials harvested as intact sheets from a mammalian source and processed to remove cellular debris advantageously retain at least a portion of and potentially all of the native collagen microarchitecture of the source extracellular matrix. This matrix of collagen fibers provides a scaffold to facilitate and support tissue ingrowth, particularly in bioactive ECM implant materials, such as porcine small intestinal submucosa or SIS (Surgisis® Biodesign™, sold by Cook Medical Incorporated of Bloomington, Indiana, USA), that are processed to retain an effective level of growth factors and other bioactive constituents from the source tissue. In this regard, when an inventive construct incorporates this sort of material, cells will invade the remodelable material upon implantation eventually leading to the generation of a newly-remodeled, functional tissue structure.

Submucosa-containing or other ECM tissue used in embodiments of the invention is preferably highly purified, for example, as described in Cook et al. U.S. Patent No. 6,206,931. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of any ECM tissue used in embodiments of the present invention.

A typical layer thickness for an as-isolated submucosa or other ECM tissue layer used in embodiments of the invention ranges from about 50 to about 250 microns when fully hydrated, more typically from about 50 to about 200 microns when fully hydrated, although isolated layers having other thicknesses may also be obtained and used. These layer thicknesses may vary with the type and age of the animal used as the tissue source. As well, these layer thicknesses may vary with the source of the tissue obtained from the animal source. In a dry state, a typical layer thickness for an as-isolated submucosa or other ECM tissue layer used in embodiments of the invention ranges from about 30 to about 160 microns when fully dry, more typically from about 30 to about 130 microns when fully dry.

Suitable bioactive agents may include one or more bioactive agents native to the source of the ECM tissue material. For example, a submucosa or other remodelable ECM tissue material may retain one or more growth factors such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in embodiments of the invention may retain other native bioactive agents such as but not limited to proteins, glycoproteins, proteoglycans, and glycosaminoglycans. For example, ECM materials may include heparin, heparin sulfate, hyaluronic acid, fibronectin, cytokines, and the like. Thus, generally speaking, a submucosa or other ECM material may retain one or more bioactive components that induce, directly or indirectly, a cellular response such as a change in cell morphology, proliferation, growth, and protein or gene expression.

Submucosa-containing or other ECM materials of embodiments of the present invention can be derived from any suitable organ or other tissue source, usually sources containing connective tissues. The ECM materials processed for use in embodiments of the invention will typically include abundant collagen, most commonly being constituted at least about 80% by weight collagen on a dry weight basis. Such naturally-derived ECM materials will for the most part include collagen fibers that are non-randomly oriented, for instance occurring as generally uniaxial or multi-axial but regularly oriented fibers. When processed to retain native bioactive factors, the ECM material can retain these factors interspersed as solids between, upon and/or within the collagen fibers. Particularly desirable naturally-derived ECM materials for use in embodiments of the invention will include significant amounts of such interspersed, non-collagenous solids that are readily ascertainable under light microscopic examination with appropriate staining. Such non-collagenous solids can constitute a significant percentage of the dry weight of the ECM material in certain inventive embodiments, for example at least about 1%, at least about 3%, and at least about 5% by weight in various embodiments of the invention.

The submucosa-containing or other ECM material used in embodiments of the present invention may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the formation of new blood vessels into the materials. Methods for measuring in vivo angiogenesis in response to biomaterial implantation have recently been developed. For example, one such method uses a subcutaneous implant model to determine the angiogenic character of a material. See C. Heeschen et al., Nature Medicine, 7(7): 833-839 (2001). When combined with a fluorescence microangiography technique, this model can provide both quantitative and qualitative measures of angiogenesis into biomaterials. C. Johnson et al., Circulation Research, 94(2): 262-268 (2004).

Further, in addition or as an alternative to the inclusion of such native bioactive components, non-native bioactive components such as those synthetically produced by recombinant technology or other methods (e.g., genetic material such as DNA), may be incorporated into an ECM material. These non-native bioactive components may be naturally-derived or recombinantly produced proteins that correspond to those natively occurring in an ECM tissue, but perhaps of a different species. These non-native bioactive components may also be drug substances. Illustrative drug substances that may be added to materials include, for example, anti-clotting agents, e.g. heparin, antibiotics, anti-inflammatory agents, thrombus-promoting substances such as blood clotting factors, e.g., thrombin, fibrinogen, and the like, and anti-proliferative agents, e.g. taxol derivatives such as paclitaxel. Such non-native bioactive components can be incorporated into and/or onto ECM material in any suitable manner, for example, by surface treatment (e.g., spraying) and/or impregnation (e.g., soaking), just to name a few. Also, these substances may be applied to the ECM material in a pre-manufacturing step, immediately prior to the procedure (e.g., by soaking the material in a solution containing a suitable antibiotic such as cefazolin), or during or after engraftment of the material in the patient.

Inventive devices can incorporate xenograft material (i.e., cross-species material, such as tissue material from a non-human donor to a human recipient), allograft material (i.e., interspecies material, with tissue material from a donor of the same species as the recipient), and/or autograft material (i.e., where the donor and the recipient are the same individual). Further, any exogenous bioactive substances incorporated into an ECM material may be from the same species of animal from which the ECM material was derived (e.g. autologous or allogenic relative to the ECM material) or may be from a different species from the ECM material source (xenogenic relative to the ECM material). In certain embodiments, ECM material will be xenogenic relative to the patient receiving the graft, and any added exogenous material(s) will be from the same species (e.g. autologous or allogenic) as the patient receiving the graft. Illustratively, human patients may be treated with xenogenic ECM materials (e.g. porcine-, bovine- or ovine-derived) that have been modified with exogenous human material(s) as described herein, those exogenous materials being naturally derived and/or recombinantly produced.

It is preferred that the attachment struts of the stent are partially coated with a parylene, to provide electrical insulation during fixation to the vessel wall. As in normal electrosurgery, temperature rise occurs in the tissue during fixation. The power is proportional to the square of the current density, so the highest temperature occurs at the surface of the stent.

Parylene is a common generic name for a variety of chemical vapor deposited poly(p-xylylene) polymers used as moisture and dielectric barriers. Among them, parylene C is the most popular due to its combination of barrier properties, cost, and other processing advantages, but parylene N and Parylene HT® can also be used. Mixtures of parylenes can also be used. ("Parylene HT" is the registered trademark of Specialty Coating Systems, Inc., Indianapolis, Indiana, USA.)

Parylene C is known for use in coating medical devices. Parylene is an excellent moisture barrier. It is the most bio-accepted coating for stents, defibrillators, pacemakers and other devices permanently implanted into the body. Parylene is a transparent polymer conformal coating that is deposited from a gas phase in a vacuum. These polymers are polycrystalline and linear in nature, possess superior barrier properties and have extreme chemical inertness. Parylene C has one chlorine group per repeat unit, i.e., it can be represented by the formula (-CH₂-C₆H₃Cl-CH₂-)ₙ. Due to its high molecular weight, parylene C has a high threshold temperature, 90 °C, and therefore has a high deposition rate, while still possessing a high degree of conformality. It can be deposited at room temperature while still possessing a high degree of conformality and uniformity and a moderate deposition rate greater than 1 nm/s in a batch process.

Parylene N is an unsubstituted polymer, which can be represented by the formula (-CH₂-C₆H₄-CH₂-)ₙ. Parylene HT® is a fluorinated polymer useful in high temperature applications, and can be represented by the formula (-CF₂-C₆H₄-CF₂-)ₙ. Parylene HT® has a lower dielectric constant than parylene N or parylene C, which is helpful in controlling the amount of current that passes at the high frequencies used in electrosurgical units.

### EXAMPLE 1 - Pig Aorta

A 12 millimeter wire stent graft, similar to that used in Cook commercial Zenith AAA stent grafts, was fastened to a pig aorta using a standard Valleylab Force 40 S electrosurgical unit with a monopolar connection. The stent graft was 12 mm in diameter, 14 mm in length, and has 7 apices on each end. The fixation was set at 70 watts of power, which was applied for 3 to 4 seconds. The aorta contracted to the wire stent and the stent set in place to the inner aortic wall. The stent was checked for adhesion and it appeared to be securely adhered.

The experiment was repeated with the other end of the same pig aorta, with the power level set at 110 watts, applied again for about 3 to 4 seconds. There was much greater affixation to the aortic wall.

### EXAMPLE 2 - Adhesion to Steak Tests

The strength of adhesion to beef steak was tested using a 14 point wire stent graft and a standard Valleylab Force 40 S electrosurgical unit with a monopolar connection, similar to those used in Example 1. A single apex pull was used except in test 1 c, in which the fixation was 2 apices; and in tests 3a to 3d, the entire stent was attached at once. In the first set of tests, different power levels were used, applied again for about 3 to 4 seconds, and the force to pull the stent free was measured. The following results were observed:

| Test | Power | Fixation | Mode | Pull Required |
|---|---|---|---|---|
| 1a | 75 | 1 Apex | Blend 1 | 61 |
| 1b | 100 | 1 Apex | Blend 1 | 70 |
| 1c | 180 | 2 Apices | Blend 1 | 81 |
| 1d | 75 | 1 Apex | Blend 1 | 67 |
| 1e | 70 | 1 Apex | Blend 1 | 76 |

In a second set of tests, the following results were observed:

| Test | Power | Fixation | Mode | Pull Required |
|---|---|---|---|---|
| 2a | 75 | 1 Apex | Blend 2 | 100 |
| 2b | 95 | 1 Apex | Blend 2 | 84 |
| 2c | 100 | 1 Apex | Blend 2 | 91 |
| 2d | 130 | 1 Apex | Blend 2 | 73 |
| 2e | 70 | 1 Apex | Blend 2 | 76 |

In a third set of tests (entire stent attached), the following results were observed:

| Test | Power | Mode | Pull Required |
|---|---|---|---|
| 3a | 75 | Blend 2 | 94 |
| 3b | 75 | Blend 1 | 102 |
| 3c | 150 | Blend 1 | 101 |
| 3d | 150 | Blend 2 | 97 |

In a fourth set of tests, the following results were observed:

| Test | Power | Mode | Pull Required |
|---|---|---|---|
| 4a | 75 | pure cut | 45 |
| 4b | 95 | pure cut | 51 |
| 4c | 90 | pure cut | 61 |
| 4d | 120 | pure cut | 81 |

While various embodiments of the invention have been described, the invention is not to be restricted except in light of the claims. Moreover, the advantages described herein are not necessarily the only advantages of embodiments of the invention and it is not necessarily expected that every embodiment of the invention will achieve all of the advantages described.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application numbers 61/720,863 and 14/066,964, from which this application claims priority, and in the abstract accompanying this application are incorporated herein by reference.

## Claims

1. An apparatus for securing an endovascular prosthesis in a body cavity, comprising:
a prosthesis introducer;
at least one expandable bio-compatible frame body disposed on the introducer, the frame body being contractible into a first shape with a smaller diameter for introduction to a vascular site and being radially expandable into a second shape having a larger diameter;
the frame body comprising at least partially electrically conductive struts for attachment to an inner wall of a body cavity; and
an insulated electrical conductor, electrically contacting at least one strut of the at least partially electrically conductive struts, the electrical conductor being connectable to radio frequency electrical power, for providing electrical energy for a sufficient time at a sufficient power to affix the strut to an inner wall of a body cavity.

2. The apparatus according to claim 1, wherein the at least partially electrically conductive struts are in contact with an inner wall of a body cavity.

3. The apparatus according to Claim 1 or 2, wherein the expandable frame body is a tubular stent comprising metal, and wherein at least one strut of the struts has at least one opening in the strut.

4. The apparatus according to Claim 3, wherein extracellular matrix material is disposed in the at least one opening.

5. The apparatus according to Claim 4, wherein the at least one opening comprises a bore having a depth extending all the way through the strut, and wherein the extracellular matrix material extends through the depth.

6. The apparatus according to any preceding Claim, wherein the at least one strut has a partial coating of an electrical insulator.

7. The apparatus according to Claim 6, wherein the partial coating of an electrical insulator comprises a parylene selected from
parylenes having a formula (-CH₂-C₆H₃Cl-CH₂-)ₙ,
parylenes having a formula (-CH₂-C₆H₄-CH₂-)ₙ,
parylenes having a formula (-CF₂-C₆H₄-CF₂-)ₙ, and
mixtures thereof.

8. The apparatus according to any preceding claim, including a radio frequency electrical power source for connection to the electrical conductor.

9. The apparatus according to any preceding claim, including means for releasing the at least one expandable bio-compatible frame body from the prosthesis introducer.

10. The apparatus according to any preceding claim, including means for expanding the at least one expandable bio-compatible frame body so that at least one at least partially electrically conductive strut contacting the electrical conductor is in contact with a body cavity.

11. The apparatus according to any preceding claim, including means for removing the electrical conductor.

12. A fixation process for fixing an at least partially electrically conductive endovascular prosthesis within a body cavity, wherein the at least partially electrically conductive endovascular prosthesis is within the body cavity in contact with a wall of the body cavity, and wherein an electrical conductor that is supplied with radio frequency electrical power is contacted to at least a portion of the at least partially electrically conductive endovascular prosthesis; the method comprising:
activating the radio frequency electrical power for sufficient time at sufficient power to affix at least a portion of the at least partially electrically conductive endovascular prosthesis to the wall of the body cavity.

13. The fixation process according to claim 12, including contacting the electrical conductor to at least a portion of the at least partially electrically conductive endovascular prosthesis.

14. The fixation process according to Claim 12 or 13, wherein the sufficient time and sufficient power accomplish a preliminary attachment of the at least partially electrically conductive endovascular prosthesis to the inner wall of the body cavity, the preliminary attachment being of such strength that the preliminary attachment can be broken without harming the wall of the body cavity in need of reinforcement to which the at least partially electrically conductive endovascular prosthesis is attached.

15. The fixation process according to any of Claims 12 to 14, wherein the body cavity is an aorta, wherein the initial sufficient time is from about 3 to about 4 seconds, the initial sufficient power is from about 70 to about 80 watts, the second sufficient time is from about 3 to about 4 seconds, and the second sufficient power is from about 95 to about 150 watts.

16. An expandable metal frame stent for securing an endovascular prosthesis in a body cavity, the metal frame stent being contractible into a first shape with a smaller diameter for introduction to a vascular site and being radially expandable into a second shape having a larger diameter;
the frame stent comprising struts for attachment to an inner wall of the body cavity;
at least one strut of the struts having at least one opening in the strut; and
extracellular matrix material being disposed in the at least one opening; and
wherein the struts have a partial coating of an electrical insulator; and
wherein the partial coating of an electrical insulator comprises a parylene selected from
parylenes having a formula (-CH₂-C₆H₃Cl-CH₂-)ₙ,
parylenes having a formula (-CH₂-C₆H₄-CH₂-)ₙ,
parylenes having a formula (-CF₂-C₆H₄-CF₂-)ₙ, and
mixtures thereof.
